# EUROPEAN PATENT SPECIFICATION

(11) **EP 2 209 442 B1**
(45) Date of publication and mention of the grant of the patent: **29.07.2015**
(21) Application number: 08846136.3
(22) Date of filing: 16.10.2008
(51) Int. Cl.: A61F 2/30, A61F 2/44, A61F 2/46

(54) **FOOTPLATE MEMBER IN AND A METHOD FOR ASSEMBLY OF A VERTEBRAL BODY REPLACEMENT DEVICE**
ENDPLATTENTEIL UND MONTAGEVERFAHREN FÜR EINE WIRBELKÖRPERPROTHESE
ÉLÉMENT DE PLAQUE DE SUPPORT À UTILISER DANS UN DISPOSITIF DE REMPLACEMENT DE CORPS VERTÉBRAL, ET PROCÉDÉ D'ASSEMBLAGE ASSOCIÉ

(30) Priority: 30.10.2007 US 928553
(43) Date of publication of application: 28.07.2010
(73) Proprietor: Aesculap AG, 78532 Tuttlingen (DE)
(72) Inventor: REFAI, Daniel, Saint Louis Missouri 63130 (US); FARRIS, Jeffrey A., Berne Indiana 46711 (US)
(74) Representative: Winter, Brandl, Fürniss, Hübner, Röss, Kaiser, Polte - Partnerschaft mbB
(86) International application number: PCT/US2008/080127
(87) International publication number: WO 2009/058576

(56) References cited:
- EP-A- 1 459 710
- EP-A- 1 867 304
- WO-A-2004/019827
- WO-A-2004/052245
- WO-A-2004/093751
- WO-A-2005/055887
- DE-A1- 4 409 392
- US-A1- 2002 068 978
- US-A1- 2003 045 877

## Description

### Technical Field

The present invention relates generally to orthopaedic and neurosurgical implants used for insertion within the spine, and more specifically, but not exclusively, concerns devices implanted within the spinal column to replace a resected, fractured or diseased vertebral body and to maintain or reestablish proper spacing between the remaining adjacent vertebral bodies.

### Background of the Invention

Damage or disease that affects the integral structure of a vertebral body within an individual's spinal column may lead to neurologic impairment with possible permanent damage to the spinal cord as well as improper neck and back alignment. Maintaining anatomic spacing within the spinal column is critical to ensuring continued functionality of the spinal cord and nerve roots and avoidance of long term serious neurological impairment.

Typically, spinal implants that are used as a spacer type of device have a fixed overall length and are implanted without the ability to adjust the degree of expansion or curvature. Recent developments of spinal spacers have resulted in devices that may be lengthened in vivo by rotary motion to match the space presented by the missing vertebral body. Problems that have been seen with these types of designs include post-placement migration attributable to the torsional forces applied to the implant during the lengthening process risking the patient to neurologic injury, the improper sizing of the implant relative to the presented clinical space, limited device access ports for height manipulation, and the lack of endplate angulation possibilities.

Document EP 1 459 710 A discloses an implant for insertion between two vertebral bodies of the spine. This document teaches an inner cylindrical element being axially movable inside an outer cylinder when a threaded ring is rotated. The ring acts on a bevel unit causing the parts to extend in length. A first disk is attached to the upper end of the inner element and a second disk to the lower edge of the outer element, facilitating an easy insertion and fixing of the device between the two vertebrae involved. Each of the disks is provided with a central bore and can also be used for the height adjustment because of their own level of thickness.

Document WO 2004/052245 A1 discloses an intervertebral implant comprising a lower implant part, which comprises a central axis and an apposition section, designed to rest against the covering surface of the adjacent lower vertebra and an upper implant part comprising a bore with an internal thread, a central axis and an apposition section, designed to rest against the covering surface of the adjacent upper vertebra. The two implant parts are secured in relation to each other against rotation about the central axis and that a threaded screw with an external thread is guided in the upper implant part and connected to the lower implant part, said external thread co-operating with the internal thread. The two implant parts and the threaded screw lie coaxially along their common central axis, said threaded screw being connected to the lower implant part so that it is axially fixed but able to rotate.

Document US 2003/045877 A1 discloses a spinal column fixation device includes a support member, a top seat fastened at the top end with the support member, and a bottom seat fastened at the bottom end with the support member. The top seat and the support member are fastened by an angle adjusting mechanism such that the angle between the top seat and the support member is adjustable. The bottom seat and the support member are fastened by an angle adjusting mechanism such that the angle between the bottom seat and the support member is adjustable.

Document WO 2005/055887 A2 discloses a height-adjustable implant for inserting between vertebrae, said implant having the following construction: a first and second sleeve part, which are rotationally fixed in coaxial alignment and interconnected in an axially displaceable manner; the first sleeve part has an internal thread; a nut is positioned coaxially in the inner space that is surrounded by the sleeve parts and is fixed to the second sleeve part so that it can rotate but be axially fixed; the nut has an external thread, which engages in the internal thread of the first sleeve part, a toothed ring, which runs coaxially with the central longitudinal axis of the sleeve parts, is located on the nut. The second sleeve part comprises a radial access opening in the vicinity of the toothed ring. According to document US 2002/068978 A1 a spacer assembly is provided for use in spinal surgeries. The spacer assembly includes a spacer having opposite ends and a side wall extending between the opposite ends and at least one end cap coupled to at least one of the opposite ends of the spacer. Each end cap includes an inner end facing the spacer, an outer end having a serrated surface, and a side wall extending between the inner and outer ends. The side wall of each end cap is formed for engagement with the side wall of the spacer to provide a mechanical connection between the end cap and the spacer.

Post published document WO 2004/019827 A1 relates to a vertebral body placeholder comprising a cylindrical inner body, which can be inserted in a telescopic manner into a coaxially arranged, sleeve-shaped outer body. The aim is to create a vertebral body placeholder that does not unwantedly penetrate spongy vertebrae. To this end, a cap is placed on a face of the inner body and/or on a face of the outer body.

It is the object of the invention to provide a footplate member for use in a vertebral body replacement device, capable to be incrementally adjusted in order to unique shapes and contours of bone surfaces engaged by the footplate, as well as a method for assembling a vertebral body replacement device.

The object of the invention is achieved by a footplate member according to claim 1 and by a method according to claims 7 and 17, respectively.

### SUMMARY OF THE INVENTION

Advancement of the state of spinal implants and the surgical management relating to the clinical presentation of missing or damaged vertebral bodies within an intact spinal column is believed desirable. The present invention satisfies the need for improvements to the vertebral space implant used to treat patients suffering from either diseased or damaged vertebral bodies by providing an in vivo adjustable vertebral body replacement device for use within a spinal column that eliminates torsional forces being applied at the implant vertebral body interface, maintains the desired optimized height, and offers 360 degrees of adjustment tool access for allowing lengthening and shortening of the device in vivo.

The present invention provides in one aspect, a footplate member for use in a vertebral body replacement device according to claim 1. The vertebral body replacement device includes a body member, a central rod and at least two end members with the central rod member being configured to be operatively associated within the body member and engage the at least two end members. The footplate member has an end surface with the end surface being configured to engage a bone surface upon implantation of the vertebral body replacement device and a sidewall that is attached to the end surface. The sidewall is configured to include an orientation mechanism that functions to align the footplate member in a certain position relative to each of the at least two end members.

The present invention provides in yet another aspect, a method for assembling a vertebral body replacement device according to claim 7.

The method includes the step of obtaining a body member that is an elongate body having an inner wall and an outer wall and includes a first end receptacle, a second end receptacle and a longitudinal axis extending between the first end receptacle and the second end receptacle. The method also includes the step of obtaining a central rod member that has a first threaded portion, a second threaded portion and a central axis extending therebetween. The central rod member is configured to be operatively associated with the body member. The method includes the step of obtaining a first end member and a second end member with the first end member being configured to be positioned within the first end receptacle of the body member to threadingly engage the first threaded portion of the central rod member when the central rod member is operatively associated with the body member, and the second end member being configured to be positioned within the second end receptacle of the body member to threadingly engage the second threaded portion of the central rod member when the central rod member is operatively associated with the body member. The method includes the further step of obtaining at least one footplate member. The at least one footplate member has an end surface with the end surface being configured to engage a bone surface upon implantation of the vertebral body replacement device. The at least one footplate member also has a mating surface attached to the end surface with the mating surface being configured to have an adjustment mechanism. The adjustment mechanism functions to align the at least one footplate member in a certain position relative to the first end member and/or the second end member. The at least one footplate member also has a locking mechanism that is configured to securely couple the at least one footplate member to the first end member and/or the second end member, thus allowing the footplate member to be modular in function. The method also includes the step of placing the central rod member within a middle chamber of the body member and operatively associating the central rod member with the body member. The method may include the step of threadingly engaging the first threaded portion of the central rod member with the first end member and threadingly engaging the second threaded portion of the central rod member with the second end member. An additional step of the method may include employing the locking mechanism to connect at least one footplate member to the first end member and/or the second end member.

The present invention provides in another aspect, a method of using at least one footplate member in a vertebral body replacement device according to claim 17. The method includes the step of obtaining a vertebral body replacement device, the vertebral body replacement device has a body member, a central rod member including a first threaded portion and a second threaded portion. The central rod member is configured to be operatively associated within the body member and a first end member and a second end member. The first end member is configured to threadingly engage the first threaded portion of the central rod member and the second end member is configured to threadingly engage the second threaded portion of the central rod member. The method also includes the step of obtaining at least one footplate member that has an end surface and a mating surface that is attached to the end surface. The mating surface is configured to have an adjustment mechanism which functions to align the at least one footplate member in a certain position relative to the first end member and/or the second end member. The at least one footplate member also has a locking mechanism that is configured to securely couple the at least one footplate member to the first end member and/or the second end member. The method includes the further step of coupling the at least one footplate member to the first end member and/or the second end member with the locking mechanism securely connecting the at least one footplate member to the first end member and/or the second end member.

Further, additional features and advantages are realized through the techniques of the present invention. Other embodiments and aspects of the invention are described in detail herein and are considered a part of the claimed invention.

### Brief Description of the Drawings

The subject matter which is regarded as the invention is particularly pointed out and distinctly claimed in the claims at the conclusion of the specification. The foregoing and other objects, features and advantages of the invention are apparent from the following detailed description taken in conjunction with the accompanying drawings in which:
FIG. 1 is a perspective, exploded view of one embodiment of a vertebral body replacement device,
FIG. 2A is a cross-sectional, side elevational view of an end member of the vertebral body replacement device of FIG. 1 taken along line 2-2, showing an inner portion with a surrounding external wall, an internal wall and an end wall with the inner portion including a centrally oriented threaded housing element configured to engage a central rod member with the end wall being oriented normal relative to the external wall,
FIG. 2B is a cross-sectional, side elevational view of an alternative embodiment of an end member, showing an inner portion with a surrounding external wall, an internal wall and an end wall with the inner portion including a centrally oriented threaded housing element configured to engage a central rod member with the end wall being oriented at an angle relative to the external wall,
FIG. 3 is a cross-sectional, side elevational view of a body member of the vertebral body replacement device of FIG. 1 taken along line 3-3, showing two receptacle ends and internal threads for engaging a support ring,
FIG. 4 is a side elevational view of a central rod member of the vertebral body replacement device of FIG. 1,
FIG. 5 is a side elevational view of the assembled vertebral body replacement device of FIG. 1, showing a superiorly positioned end member and an inferiorly positioned end member extended away from the body member,
FIG. 6 is a perspective view of the vertebral body replacement device of FIG. 1, with a tool inserted through a tool port hole and in operable position with the central rod member,
FIG. 7 is a side elevational view of the vertebral body replacement device of FIG. 1, shown disposed within a space between two vertebral bodies within a spinal column prior to the translational movement of the superiorly positioned end member and the inferiorly positioned end member,
FIG. 8 is a side elevational view of the vertebral body replacement device of FIG. 1, shown positioned between two vertebral bodies with the superiorly positioned end member and the inferiorly positioned end member extended to maintain a desired space within a spinal column,
FIG. 9 is a perspective view of an alternative embodiment of a vertebral body replacement device, with a superiorly positioned, detachable footplate member and an inferiorly positioned, detachable footplate member shown prior to being coupled to the superiorly positioned end member and an inferiorly positioned end member, respectively, in accordance with an aspect of the present invention;
FIG. 10A is a side elevational view of a detachable footplate member of the vertebral body replacement device of FIG. 9, showing an end surface being oriented normal relative to a sidewall, in accordance with an aspect of the present invention; and
FIG. 10B is a side elevational view of an alternative embodiment of a detachable footplate member used with the vertebral body replacement device of FIG. 9, showing the end surface being oriented at an angle relative to the sidewall, in accordance with an aspect of the present invention.

### Best Mode for Carrying Out The Invention

Generally stated, disclosed herein is a vertebral body replacement device or vertebral spacer that typically includes a body member, a central rod member, a support ring, two end members and at least one footplate member. As used herein, the terms "vertebral body replacement device" and "vertebral spacer" may be used interchangeable as they essentially describe the same type of implant device. Further, described herein is a surgical method for using the vertebral body replacement device to maintain a space between two vertebral bodies within a patient suffering from a diseased or damaged spinal column.

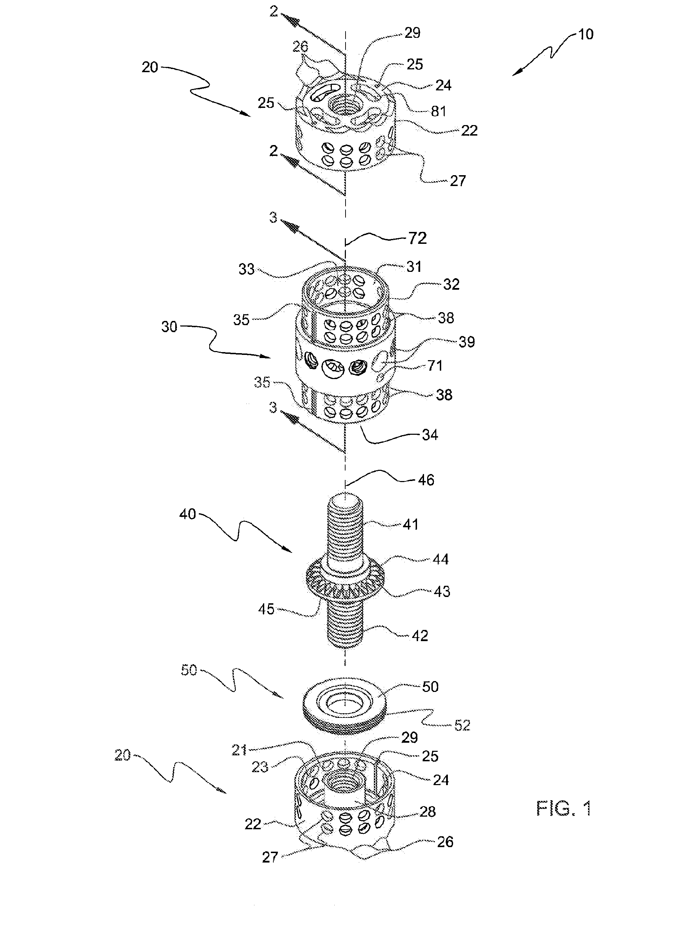

As depicted in FIG. 1, the general arrangement of a vertebral body replacement device 10, includes a body member 30, at least two end members 20, a central rod member 40 and a support ring 50. In this detailed description and the following claims, the words proximal, distal, anterior, posterior, medial, lateral, superior and inferior are defined by their standard usage for indicating a particular part of a bone or prosthesis according to the relative disposition of the natural bone or directional terms of reference. For example, "proximal" means the portion of a prosthesis nearest the torso, while "distal" indicates the portion of the prosthesis farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure.

With reference to FIG. 1, vertebral body replacement device 10 includes body member 30, at least two end members 20 positioned superior and inferior relative to body member 30, a central rod member 40 for placement within body member 30 and support ring 50 that is configured to contact and secure central rod member 40 within body member 30.

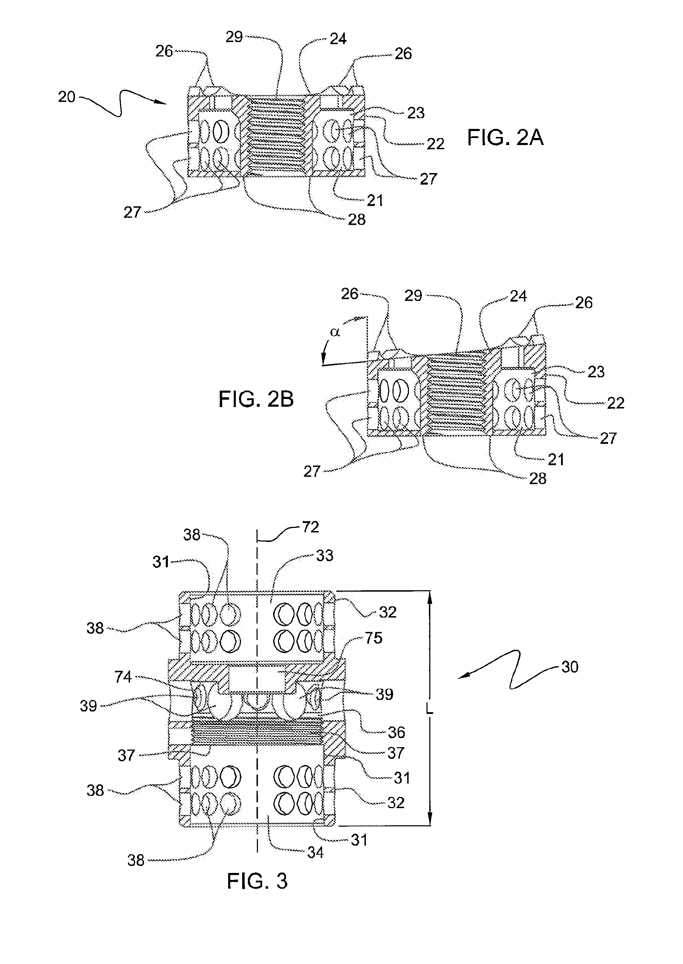

Exhibited in FIG. 1, body member 30 also includes an inner wall 31 and an outer wall 32, at least one hole 38 extending from outer wall 32 through inner wall 31. Further, body member 30 has at least one anti-rotational rib 35 disposed on and extending for substantially the entire length of outer wall 32. At least one rib 35 is oriented in a superior to inferior direction relative to body member 30 and substantially parallel to a longitudinal axis 72 of body member 30. At least one hole 38 is used for the placement of bone graft or other biocompatible material that will facilitate bone fusion to occur in vivo following implantation of the device. It should be understood to those skilled in the art that body member 30 may be available to the operating surgeon in various outside diameter sizes and longitudinal lengths L (see FIG. 3). Having multiple sized body members 30 as part of an implant system allows the operating surgeon to use vertebral body replacement device 10 in various levels or segments of the spine (i.e., smaller sizes in the cervical spine, medium sizes in the thoracic spine and larger sizes in the lumbar spine).

As shown in the cross-sectional view of FIG. 3, body member 30 further includes a first or superiorly positioned end receptacle 33 and a second or inferiorly positioned end receptacle 34 with longitudinal axis 72 extending between these two structures within elongate body member 30. A middle chamber 36 is defined by inner wall 31 and is bound superiorly by first end receptacle 33 and inferiorly by second end receptacle 34. At least one tool port hole 39 extends into middle chamber 36 through outer wall 32 and inner wall 31. In addition, inner wall 31 of middle chamber 36 includes a set of internal threads 37 positioned in the bottom portion of middle chamber 36. Internal threads are sized and configured to threadingly engage the external threads 52 of support ring 50 (not shown). A ceiling surface 74 bounds the superior portion of middle chamber 36 with a centralized opening 75 positioned through ceiling surface 74. Although not shown, when vertebral body replacement device 10 is fully assembled and in use, central rod member 40 is operatively associated with body member 30 by being configured to allow for a superior threaded portion 41 of central rod member 40 to pass through centralized opening 75 resulting in a collar element 47 of central rod member 40 contacting ceiling surface 74. Following placement of superior threaded portion 41 of central rod member 40 through centralized opening 75, central rod member 40 is moveably secured within middle chamber 36 by threadingly coupling support ring 50 to internal threads 37 of middle chamber 36 resulting in a bearing surface 51 of support ring 50 making pressing contact with a support surface 45 of central rod member 40. Body member 30 further includes at least one locking pin hole 71 (as seen in FIG. 1) that passes through outer wall 32 and inner wall 31 into middle chamber 36. Although not shown, following final placement and adjustment of assembled vertebral body replacement device 10, a corresponding threaded pin or bolt may screw into at least one locking pin hole 71 resulting in central rod member 40 being secured in position, fixing the overall length of vertebral body replacement 10.

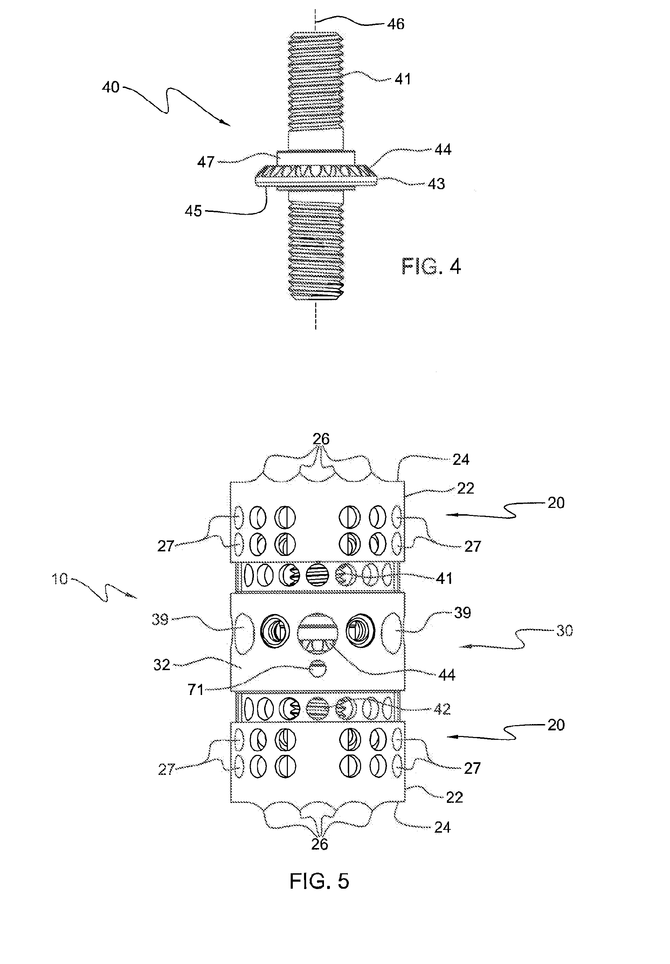

FIGS. 1 and 4 show central rod member 40 having first or superior threaded portion 41 and a second or inferior threaded portion 42 with the two threaded portions having opposing thread configurations. This means that when first threaded portion 41 is constructed with right-handed threads, second threaded portion 42 is constructed with left-handed threads. It should be understood to those skilled in the art that the vice-versa thread configuration is also contemplated. Central rod member 40 further includes a central axis 46 that passes from first threaded portion 41 to second threaded portion 42 with a gear wheel portion 43 being positioned intermediate first threaded portion 41 and second threaded portion 42. Gear wheel portion 43 is generally constructed with a toothed face surface 44, the plane of toothed face surface 44 being oriented substantially perpendicular to central axis 46. Collar element 47 is positioned adjacent to tooth face surface 44 to ensure proper external access of tooth face surface 44 within middle chamber 36 following assembly of vertebral body replacement device 10. Further, gear wheel portion 43 includes support surface 45 that is located on the inferior aspect or underside of gear wheel portion 43. Similar to that described for toothed wheel surface 44, the plane of support surface 45 is correspondingly oriented substantially perpendicular to central axis 46. As explained previously, support surface 45 will contact and slidingly articulate with bearing surface 51 of support ring 50 (see FIG. 1) when vertebral body replacement device 10 is assembled and in use. Gear wheel portion 43 is integral to central rod member 40 and is positioned so that when gear wheel portion 43 is moved about its rotational axis, first threaded portion 41 and second threaded portion 42 will also rotate because gear wheel portion 43 axis of rotation is coaxial with central axis 46.

FIGS. 1, 2A and 2B depict end member 20. Vertebral body replacement device 10 includes in its construct at least two end members 20, with the first one end member 20 being positioned superiorly relative to body member 30 and the second end member 20 being positioned inferiorly relative to body member 30. In operation, superiorly positioned first end member 20 is aligned and concentric with first end receptacle 33 so that when first end member 20 moves relative to body member 30, an internal wall 23 of end member 20 is continuously positioned adjacent to outer wall 32 of first end receptacle 33. The same operational relationship occurs with inferiorly positioned second end member 20 as it will be aligned and concentric with second end receptacle 34 so that when second end member 20 moves relative to body member 30, internal wall 23 of end member 20 is continuously positioned adjacent to outer wall 32 of second end receptacle 34.

As seen in FIGS. 2A and 2B, end member includes an inner portion 21 that is bounded by internal wall 23 and a centrally positioned threaded housing element 28. Threaded housing element 28 is constructed with internal threads 29 that may extend the full length of threaded housing element 28. Internal threads 29 are configured to correspondingly threadingly engage threaded portions 41, 42 of central rod member 40 upon assembly of vertebral body replacement device 10. Although not shown in FIGS. 2A and 2B, internal wall 23 also includes at least one channel 25 (see FIG. 1) with at least one channel 25 being oriented substantially vertical and is sized to receive corresponding at least one anti-rotational rib 35 of body member 30 when vertebral body replacement device 10 is assembled.

As further shown in the cross-sectional views of FIGS. 2A and 2B, end member 20 has an external wall 22, through which at least one hole 27 passes to adjacent internal wall 23. At least one hole 27 is sized to allow for the placement of bone graft material and other biocompatible materials for the purpose of facilitating a bone fusion bed following implantation.

Additionally, as seen in FIGS. 1 and 2A, end wall 24 functions to cap or bound inner portion 21 at one end of end member 20. End wall 24 is integrally coupled to threaded housing element 28 and generally includes at least one projection 26 or engagement element that extends in an outward direction from the outer surface of end wall 24. At least one projection 26 may be configured as a tooth-like body (as shown in FIGS. 1, 2A, 2B, and 5) although other shaped projections or engagement elements are contemplated including, but not limited to spikes, pegs, grids, fingers and posts. At least one projection 26 is sized to allow for operative engagement with the adjacent vertebral body, more specifically with the anatomic end plate of the vertebral body to provide adequate fixation post-implantation and to withstand any torsional loads that may be applied to end member 20 following implantation and during the lengthening procedure of vertebral body replacement device 10.

Cross-section view of FIG. 2A shows, end wall 24 being oriented perpendicular or normal relative to external wall 22. FIG. 2B shows an alternative embodiment of end member 20 with end wall 24 being oriented at an angle α and relative to external wall 22. Having end wall 24 being angled provides the operating surgeon with the ability to treat clinically, lordotic and kyphotic deformities. It should be well understood to those skilled in the art that end member 20 will be offered in a wide range of degrees of angulations in varying increments from 0° to 20°, thereby providing the operating surgeon with the ability to precisely treat any deformity presented during a surgical procedure.

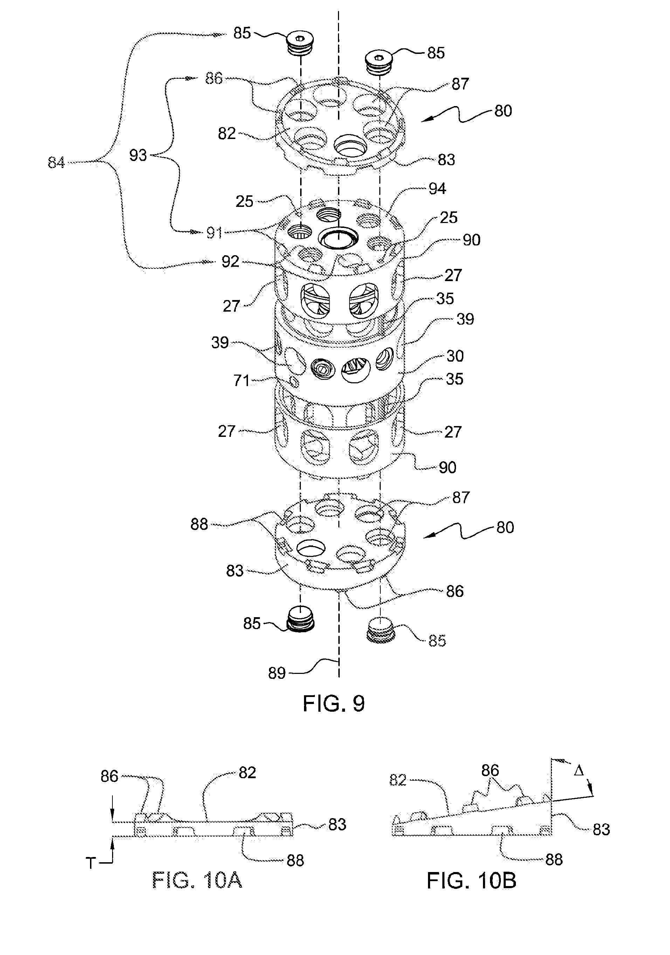

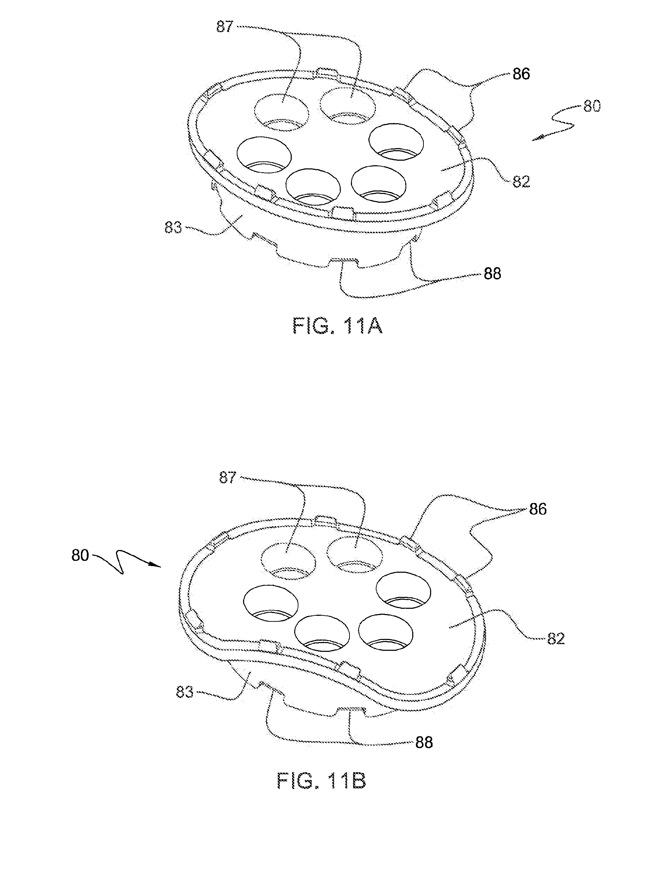

As shown in FIG. 9, vertebral body replacement device 10 may include an alternative embodiment of end member 90, with end wall 94 being configured to couple a footplate member 80 according to the invention. End wall 94 may further include at least one alignment tab 91 that functions to orient footplate member 80 in the preferred position relative to a central axis 89 and end member 90, and a vertebral body following implantation. As seen in FIGS. 11A and 11B, it is contemplated that footplate member 80 will be available in a plurality of various circular, non-circular and polygonal outer profile shapes, (i.e., circular as shown in FIG. 9, oval as shown in FIG. 11A, kidney as shown in FIG. 11B or oblong (not shown)) and sizes. It is further contemplated that footplate member 80 will be available in varying thicknesses or heights T as seen in FIG. 10A. Having a kit or implant system that includes a range of various sized heights, shapes, sizes and angled footplate members 80 provides the operating surgeon with multiple choices to maximum bone coverage, spine alignment and resulting stability of the device relative to the adjacent vertebral body following implantation.

As shown in FIG. 10A, an end surface 82 may be configured in a neutral or normal orientation relative to a sidewall or mating surface 83 of footplate member 80. Alternatively, FIG. 10B shows footplate member 80 having end surface 82 being angled (angle Δ) relative to sidewall or mating surface 83. As discussed above, it is contemplated that the operating surgeon will be provided with a plurality of footplate members 80 each having a different angle, with angulation ranging from 0° to 20°. Having such a wide range of incrementally angled footplate members 80 available will provide the operating surgeon with the ability to customize the vertebral body replacement device 10 during the operative procedure to meet the presented clinical deformity. Although shown with a circular perimeter geometry in FIG. 9, as described previously it should be understood to those skilled in the art that both neutral and angled footplate members 80 will be constructed in multiple outer profile geometric shapes, sizes and overall thickness T, again to provide the operating surgeon with the ability to maximize bone support post-implantation. Footplate member 80 may be modular in design, thereby allowing the operating surgeon to mix and match and interchange footplate members 80 with end member 90. This is accomplished by securely attaching and allowing detachment of footplate member 80 from end wall 94 of end member 90 by use of a locking mechanism 84. For example purposes only, as shown, locking mechanism 84 may consist of at least one locking screw 85 that passes through a hole 87 in end surface 82 to engage corresponding threaded holes 92 in end wall 94. Further, it should be understood to those skilled in the art that various other low-profile locking or securement mechanisms may also be used for this purpose including, but not limited to lock pins, bolts, and press fit pins.

As described above, it is contemplated that footplate member 80 will also include at least one projection 86 or engagement element that extends outwardly from the end surface 82. At least one projection 86 may be configured as a tooth-like projection (as shown in FIGS. 9, 10A, and 10B,) although other shaped engagement elements are contemplated, including but not limited to, spikes, pegs, grids, figures and posts. End surface 82 may be treated or coated with certain materials to facilitate bio-ingrowth with the adjacent vertebral body following implantation. Additionally, end surface 82 may also undergo a process or treatment that results in end surface 82 having nano-sized or micron-sized surface features.

As seen in FIG. 9, footplate member 80 has an orientation or adjustment mechanism 93 that includes alignment slots 88 that are positioned along mating surface or sidewall 83. Slots 88 will slidingly engage corresponding tabs 91 positioned around the peripheral of end member 90. Orientation or adjustment mechanism 93 functions to facilitate the positioning of footplate member 80 relative to end member 90 and more specifically to end wall 94 and ultimately when implanting, the adjacent vertebral body within the spinal column of a patient. Footplate member 80 when moved is typically rotated or moved relative to central axis 89 and end member 90. It is contemplated further that an alternative embodiment of orientation or adjustment mechanism 93 may be positioned in the more central portions of end wall 94 and end surface 82, respectively.

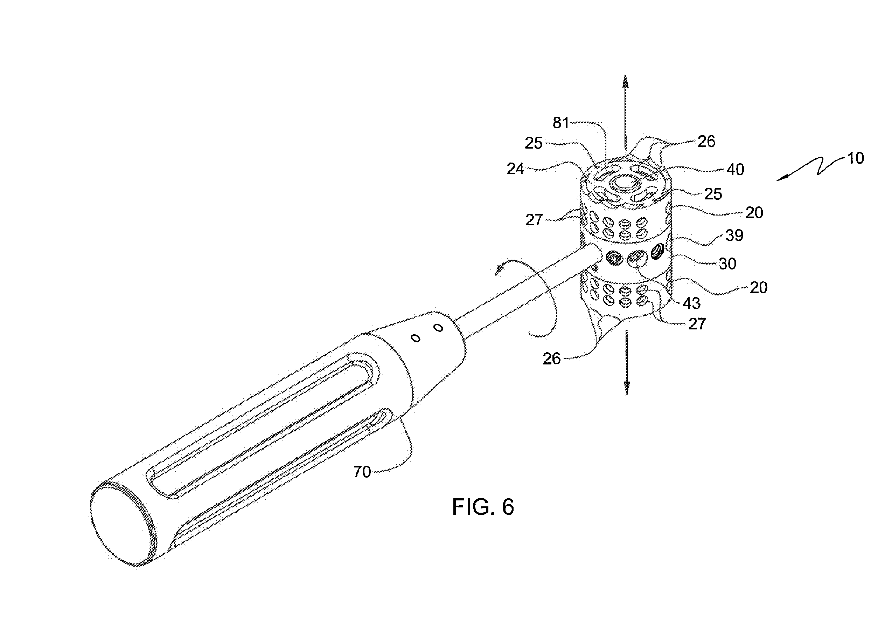

Following the assembly of vertebral body replacement device 10, superiorly positioned or first end member 20 and inferiorly positioned or second end member 20 are both positioned with each respective inner portion 21 and threaded housing element 28 within first end receptacle 33 and second end receptacle 34, respectively. As shown in FIG. 6, first end member 20 and second end member 20 may be simultaneously extended or retracted in an axial direction relative to body member 30 resulting in either the lengthening or shortening of the over-all length of vertebral body replacement device 10 by inserting a tool 70 through tool port hole 39 to engage the gear shaped tip (not shown) of tool 70 with tooth faced surface 44 of gear wheel portion 43 of central rod member 40. In operation, tool 70 is rotated causing gear wheel portion 43 to rotate resulting in first and second threaded portions 41, 42 rotating about central axis 46. When assembled, threaded housing element 28 of first and second end members 20 are threaded onto first and second threaded portions 41, 42 of central rod member 40 respectively, with at least one channel 25 of first and second end members 20 also engaging at least one anti-rotational rib 35 positioned on outer wall 32 of first and second end receptacles 33, 34, respectively. Functionally, the engagement of at least one channel 25 of first and second end members 20 with at least one rib 35 of body member 30 prohibits rotational movement of the first and second end members 20 when tool 70 is turned, thus resulting in first and second end members 20 simultaneously advancing or moving in opposing axial directions relative to body member 30 for a maximum distance equal to the thread length of first and second thread portions 41, 42 of central rod member 40. As discussed above, the bidirectional axial motion of the first end and second end members 20 is caused by the opposing threads (i.e., right-handed and left handed threads) of the respective first and second threaded portion 41, 42 of the central rod member 40. Operationally, central rod member 40 converts the rotational motion of tool 70 and gear wheel portion 43 into corresponding axial or linear movement of first and second end members 20, with the mating of channel 25 and rib 35 substantially prohibiting any rotational movement of two end members 20 relative to longitudinal axis 72 and the adjacent vertebrae, thus eliminating torsional forces being applied to the end member- vertebral body interface. For example purposes, FIG. 5 shows an assembled vertebral body replacement device 10 following partial simultaneous movement of first and second end members 20 as describe above.

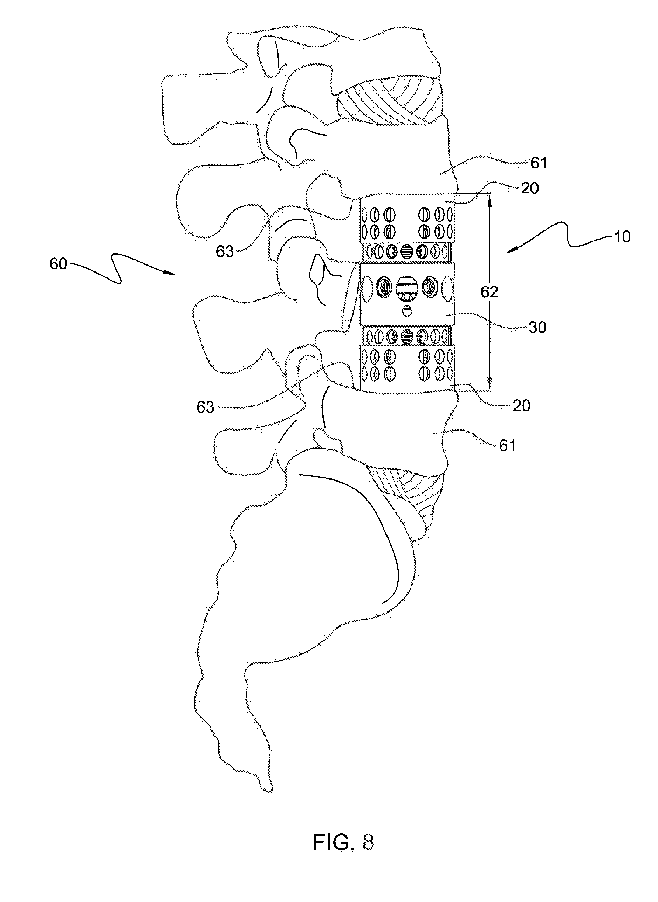

FIG. 8 shows assembled vertebral body replacement device 10 positioned within a space between two vertebral bodies following simultaneous movement of first and second end members 20 in the manner described above, resulting in intimate contact between an adjacent vertebral body and at least one projection 26 extending from end wall 24, or alternatively, projection 86 of footplate member 80 (not shown). A resultant compressive force is applied by each end member 20 (or footplate member 80) against the contacted vertebral body to maintain the desired anatomic spacing.

The surgical technique for implantation of a vertebral body replacement device is well known in the art, including the appropriate surgical exposure and dissection techniques. The method includes, obtaining a vertebral body replacement device 10 that may include body member 30, central rod member 40 that has two threaded portions 41, 42 and is configured to be operatively associated within body member 30 and first and second end members 20 that are configured to threadingly engage the two threaded portions 41, 42 of central rod member 40. As discussed above, body member 30 and end members 20 are further configured to inhibit rotational movement of two end members 20 following assembly and positioning of vertebral body replacement device 10 within a space within a spinal column with both end members 20 engaging respective vertebral bodies when central rod member 40 is rotationally actuated, thus causing two end members 20 to move in opposing axial directions relative to body member 30. Upon such movement, two end members 20 will apply a force to the two adjacent vertebral bodies within the spinal column. It should be understood that all of the above noted device components and respective elements include the same structural and functionality characteristics as described previously herein.

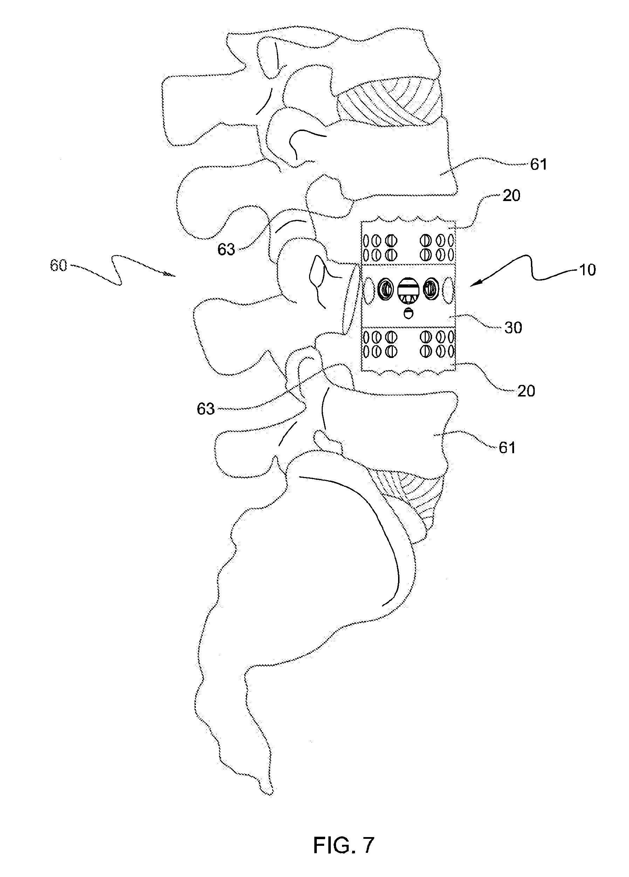

As seen in FIG. 7, the method may further include the step of positioning vertebral body replacement device 10 between two vertebral bodies within a patient's spinal column. The surgical method may also include the step of simultaneously operatively moving in opposing directions both end members 20 relative to body member 30 to produce a force against the two respective adjacent vertebral bodies for the purpose of maintaining a space between the two vertebral bodies within the spinal column as shown in FIG. 8. Although not shown, the method may further include the step of engaging tool 70 with central rod member 40 through tool portal hole 39, whereby rotary motion of tool 70 is converted into opposing axial movement of two respective end members 20 relative to body member 30 causing two end members 20 to come in contact and apply a force to the adjacent vertebral bodies, thereby maintaining the space between these two vertebral bodies. The method also may include the step of securely coupling to body member 30 a lock pin through lock pin hole 71 following finalization of the length adjustment procedure to ensure securement of two end members 20 relative to body member 30 and central rod member 40.

It should be understood by those skilled in the art that the surgical method described herein may also include, alternatively, using modular footplate member 80 that has been coupled to alternative embodiment end member 90 which has been more fully described above. The sequence of implantation of vertebral body replacement device 10 as described herein may be different depending upon the given clinical situation and whether footplate members 80 are attached on the "back table" prior to the complete assembly of vertebral body replacement device 10 or within the operative site. It is contemplated that footplate member 80 would be oriented relative to end member 80 and a vertebral body within the spine to facilitate and maximize bone contact and stability. Final securement and positioning of footplate member 80 to end member 80 is dependent upon the operating surgeon achieving adequate alignment during trial implantation. Once this has been achieved, orientation or adjustment mechanism 93 will be used to finalize the position of footplate member 80 relative to the vertebral body, with locking mechanism 84 being actuated to secure footplate member 80 to end member 90. The sequence of device orientation, assembly and securement will be at the discretion of the operating surgeon and will vary depending upon the preference of the operating surgeon in combination with the clinical needs of the patient.

It is further contemplated that an implant system comprised of various cross-sectional sizes, cross-sectional polygonal and circular/oval shapes and longitudinal lengths of body members 30, end members and footplate member 80 will be available as a kit. This will allow the operating surgeon to pick and choose the separate member components to assemble vertebral body replacement device 10 that best fits into a certain spinal segment or to address an anatomical deformity presented in a patient. It should be understood by those skilled in the art that each shaped and dimensioned member provided will function in the same manner as described previously herein with central rod member 40 and supporting ring 50.

In one example, a method for assembling vertebral body replacement device 10 includes obtaining body member 30 that is an elongate body having inner wall 31 and outer wall 32 and includes first end receptacle 33, second end receptacle 34 and longitudinal axis 72 extending between first end receptacle 33 and second end receptacle 34. The method also includes obtaining central rod member 40 that has first threaded portion 41, second threaded portion 42 and central axis 46 extending therebetween. Central rod member 40 is configured to be operatively associated with body member 30. The method includes obtaining first end member 20 and second end member 20 with first end member 20 being configured to be positioned within first end receptacle 33 of body member 30 to threadingly engage first threaded portion 41 of central rod member 40 when central rod member 40 is operatively associated with body member 30 and second end member 20 being configured to be positioned within second end receptacle 34 of body member 30 to threadingly engage second threaded portion 42 of central rod member 40 when central rod member 40 is operatively associated with body member 30. The further method includes obtaining at least one footplate member 80 which has end surface 82 with end surface 82 being configured to engage a bone surface upon implantation of the vertebral body replacement device. At least one footplate member 80 also has a sidewall adjustment or mating surface 83 attached to end surface 82 with sidewall 83 being configured to have orientation adjustment mechanism 93. The orientation adjustment mechanism 93 functions to align at least one footplate member 80 in a certain position relative to first end member 20 and/or the second end member 20 and a vertebral body. At least one footplate member 80 also has locking mechanism 84 that is configured to securely couple at least one footplate member 80 to the first end member 20 and/or the second end member 20, thus allowing the at least one footplate member 80 to be modular in function. The method also includes placing central rod member 40 within middle chamber 36 of the body member 30 and operatively associating central rod member 40 with body member 30. The method may include threadingly engaging first threaded portion 41 of central rod member 40 with first end member 20 and threadingly engaging second threaded portion 42 of central rod member 40 with the second end member 20. An additional step of the method may also include employing locking mechanism 84 to connect at least one footplate member 80 to first end member 20 and/or second end member 20.

In another example, a method of using at least one footplate 80 member in a vertebral body replacement device 10 includes obtaining vertebral body replacement device 10 that includes body member 30, central rod member 40 including first threaded portion 41 and second threaded portion 42. Central rod member 40 is configured to be operatively associated within body member 30 and first end member 20 and second end member 20. First end member 20 is configured to threadingly engage first threaded portion 41 of central rod member 40 and second end member 20 is configured to threadingly engage second threaded portion 42 of central rod member 40. The method also includes obtaining at least one footplate member 80 or mating surface 83 that has end surface 82 and sidewall or mating surface 83 that is attached to end surface 82. Sidewall 83 is configured to have orientation adjustment mechanism 93 which functions to align the at least one footplate member 80 in a certain position relative to first end member 20 and/or second end member 20. At least one footplate member 80 also has locking mechanism 84 that is configured to securely couple the at least one footplate member 80 to first end member 20 and/or second end member 20. The method further includes coupling at least one footplate member 80 to first end member 20 and/or second end member 20 with locking mechanism 84 securely connecting the at least one footplate member 80 to first end member 20 and/or second end member 20.

Although the preferred embodiments have been depicted and described in detail herein, it will be apparent to those skilled in the relevant art that various modifications, additions and substitutions can be made without departing from the scope of the following claims.

## Claims

1. A footplate member (80) for use in a vertebral body replacement device (10), the footplate member (80), **characterized by** comprising:
an end surface (82), wherein the end surface (82) is configured to engage a bone surface upon implantation of the vertebral body replacement device within a spinal column, the end surface (82) having a center section that is solid, and a plurality of holes (87) surrounding the center section of end surface (82) and extending trough footplate member (80);
a sidewall (83) connected to the end surface (82) with the end surface (82) spanning an entire area bounded by the sidewall (83), and with the sidewall (83) being configured to have an adjustment mechanism (93), wherein the adjustment mechanism facilitates the positioning of the footplate member (80) relative to an end member (90) of the vertebral body replacement device, the adjustment mechanism (93) comprising a plurality of alignment slots (88) positioned along sidewall (83), wherein each alignment slot (88) is sized to mate with one of a plurality of tabs (91) on said end member of the vertebral body replacement device, thereby orienting the footplate member (80) in a certain position relative to said end member (90) of the vertebral body replacement device;
the plurality of holes (87) extending through footplate member (80) between the end surface (82) and the sidewall (83); and
a locking mechanism (84), wherein the locking mechanism (84) is configured to couple the footplate member (80) to the end member (90) through one of the plurality of holes (87) prior to implantation of the vertebral body replacement device within the spinal column.

2. The footplate member (80) of claim 1, wherein the locking mechanism (84) comprises a set screw (85) and an opening, wherein the set screw is configured to pass through one of the plurality of holes (87) within the footplate member and to be received into an opening (92) disposed within the end member (90), the set screw when threadingly engaged within the opening securely couples the footplate member to the end member.

3. The footplate member (80) of claim 1, wherein the end surface (82) comprises a plurality of projections (86), each of the plurality of projections is configured to extend away from the end surface, thereby to engage and secure the footplate member (80) against an adjacent vertebral body upon implantation of the vertebral body replacement device.

4. The footplate member (80) of claim 1, wherein the end surface (82) of the footplate member is oriented substantially normal or at an angle relative to the sidewall (83).

5. The footplate member (80) of claim 1, wherein an outer profile of the end surface (82) is at least one of a polygonal shape, a non-circular shape and a circular shape, wherein the shape of the outer profile of the end surface is selected to correspond the shape of the adjacent vertebral body following implantation of the vertebral body replacement device within the spinal column.

6. The footplate member (90) of claim 1, wherein the end surface (82) of the footplate member comprises a plurality of surface features, wherein the surface features are micron-sized or nano-sized surface features.

7. The footplate member (80) of claim 1, wherein the end surface (82) of the footplate member is configured and coated with a substance to facilitate bio-ingrowth of the end surface to an adjacent vertebral body following implantation of the vertebral body replacement device within a spinal column.

8. A method for assembling a vertebral body replacement device (10), the method **characterized by** comprising:
obtaining a body member (30), wherein the body member is an elongate body having an inner wall (31) and an outer wall (32), and comprising a first end receptacle (33), a second end receptacle (34) and a longitudinal axis (72) extending between the first end receptacle and the second end receptacle thereof;
obtaining a central rod member (40) having a first threaded portion (41), a second threaded portion (42) and a central axis (46) extending therebetween, the central rod member being configured to be operatively associated with the body member (30);
obtaining a first end member (20) and a second end member (20), wherein the first end member is configured to be positioned within the first end receptacle (33) of the body member (30) to threadingly engage the first threaded portion (41) of the central rod member (40) when the central rod member is operatively associated with the body member, and the second end member (20) is configured to be positioned within the second end receptacle (34) of the body member to threadingly engage the second threaded portion (42) of the central rod member when the central rod member is operatively associated with the body member; and
obtaining at least one footplate member (80), wherein the at least one footplate member comprises:
an end surface (82), wherein the end surface (82) is configured to engage a bone surface upon implantation of the vertebral body replacement device (10) within a spinal column, the end surface (82) having a center section that is solid, and a plurality of holes (87) surrounding the center section of end surface (82) and extending through footplate member (80);
a sidewall (83) connected to the end surface (82) with the end surface (82) spanning an entire area bounded by the sidewall (83), and with being configured to have an adjustment mechanism (93), wherein the adjustment mechanism facilitates the positioning of the at least one footplate member (80) relative to at least one of the first end member (20) and second end member (20), the adjustment mechanism (93) comprising a plurality of alignment slots (88) positioned along sidewall (83), wherein each alignment slot (88) is sized to mate with one of a plurality of tabs (91) on at least one of the first end member (20) and second end member (20) thereby orienting the at least one footplate member (80) in a certain position relative to at least one of the first end member (20) and second end member (20); and
a locking mechanism (84), wherein the locking mechanism is configured to couple the at least one footplate member (80) to at least one of the first end member (20) and second end member (20), thereby allowing the footplate to be modular;
placing the central rod member (40) within a middle chamber (36) of the body member (30) and operatively associating the central rod member with the body member;
threadingly engaging the first threaded portion (41) of the central rod member (40) with the first end member (20) and threadingly engaging the second threaded portion (42) of the central rod member with the second end member (20);
employing the adjustment mechanism (93) to orient the at least one footplate member (80) in a certain position relative to said end member (90); and
employing the locking mechanism (84) to connect the at least one footplate member (80) to at least one of the first end member (20) and second end member (20) prior to implanting the vertebral body replacement device within a spinal column.

9. The method of claim 8, wherein the obtaining at least one footplate member (80) further comprises the adjustment mechanism (93) comprising a plurality of tabs (91) being disposed on an end wall of each of the first end member (20) and the second end member (20) and the corresponding alignment slots (88) being positioned along the sidewall (83) of the footplate member (80), wherein each alignment slot (88) is sized to mate with one of the plurality of tabs (91) and, thereby orient the footplate member (80) in a certain position relative to the end wall of at least one of the first end member (20) and second end member (20) and a vertebral body following implantation of the vertebral body replacement device.

10. The method of claim 8, wherein the end surface of the at least one footplate member (80) is oriented substantially normal or at an angle relative to the sidewall (83).

11. The method of claim 8, wherein an outer profile of the end surface of the at least one footplate member (80) is at least one of a polygonal shape, a non-circular shape and a circular shape, wherein the shape of the outer profile of the end surface is selected to correspond the shape of the adjacent vertebral body following implantation of the vertebral body replacement device within a spinal column.

12. The method of claim 8, wherein the end surface of the at least one footplate member (80) comprises a plurality of surface features with the surface features being micron-sized or nano-sized.

13. The method of claim 8, wherein the end surface of the at least one footplate member (80) is configured and coated with a substance to facilitate bio-ingrowth of the end surface to an adjacent vertebral body following implantation of the vertebral body replacement device within a spinal column.

14. The method of claim 8, further comprising obtaining a support ring (50), wherein the support ring has a bearing surface (51), and is configured to threadingly engage the inner wall of the body member (30), thereby allowing the bearing surface to contact the central rod member (40) when the central rod member is operatively positioned within the body member.

15. The method of claim 14, wherein the obtaining a central rod member (40) further comprises a gear wheel portion (43), the gear wheel portion having a toothed face surface (44) and a support surface (45), the support surface of the gear wheel portion being configured to contact the bearing surface of the support ring (50) when the central rod member is operatively positioned within the body member.

16. The method of claim 15, wherein the central rod member (40) comprises a central axis (46) extending between the first threaded portion (41) and the second threaded portion (42) thereof, and wherein the rotational axis of the gear wheel portion (43) is substantially coaxial to the central axis of the central rod member, thereby when the gear wheel portion is rotated about the rotational axis the first and second threaded portions correspondingly rotate about the central axis (46) of the central rod member.

17. A method of using at least one footplate member (80) in a vertebral body replacement device (10), the method **characterized by** comprising:
obtaining a vertebral body replacement device (10), the vertebral body replacement device including a body member (30), a central rod member (40) having a first threaded portion (41) and a second threaded portion (42), the central rod member being configured to be operatively associated within the body member and a first end member (20) and a second end member (20), the first end member being configured to threadingly engage the first threaded portion (41) of the central rod member (40) and the second end member being configured to threadingly engage the second threaded portion (42) of the central rod member;
obtaining at least one footplate member (80), the at least one footplate member comprising an end surface (82), the end surface (82) having a center section that is solid, and a plurality of holes (87) surrounding the center section of end surface (82) and extending through footplate member (80), and a sidewall (83) connected to the end surface, with the sidewall being configured to have an adjustment mechanism (93), wherein the adjustment mechanism facilitates the positioning of the at least one footplate member (80) relative to at least one of the first end member (20) and second end member (20), and a locking mechanism (84), wherein the locking mechanism (84) is configured to couple the at least one footplate member (80) to at least one of the first end member (20) and second end member (20), the adjustment mechanism (93) comprising a plurality of alignment slots (88) positioned along sidewall (83), wherein each alignment slot (88) is sized to mate with one of a plurality of tabs (91) on at least one of the first end member (20) and second end member (20) thereby orienting the at least one footplate member (80) in a certain position relative to at least one of the first end member (20) and second end member (20); and
coupling the at least one footplate member (80) to at least one of the first end member (20) and second end member (20), the locking mechanism securely connecting the at least one footplate member to at least one of the first end member and second end member.

## Patentansprüche

1. Endplattenbauteil (80) für den Gebrauch in einer Wirbelkörperersatzvorrichtung (10), wobei das Endplattenbauteil (80) **gekennzeichnet ist durch** das Aufweisen:
einer Endoberfläche (82), wobei die Endoberfläche (82) ausgebildet ist, um in eine Knochenoberfläche bei einer Implantation der Wirbelkörperersatzvorrichtung innerhalb einer Wirbelsäule einzugreifen, wobei die Endoberfläche (82) einen Mittelabschnitt, der massiv ist, und eine Mehrzahl an Löchern (87) hat, welche den Mittelabschnitt der Endoberfläche (82) umgeben und sich **durch** das Endplattenbauteil (80) erstrecken;
einer Seitenwand (83), die mit der Endoberfläche (82) verbunden ist, wobei die Endoberfläche (82) ein gesamtes Gebiet aufspannt, welches **durch** die Seitenwand (83) begrenzt ist, und wobei die Seitenwand (83) ausgebildet ist, um einen Einstellungsmechanismus (93) zu haben, wobei der Einstellungsmechanismus die Positionierung des Endplattenbauteils (80) relativ zu einem Endbauteil (90) der Wirbelkörperersatzvorrichtung erleichtert, wobei der Einstellungsmechanismus (93) eine Mehrzahl an Ausrichtungsschlitzen (88) aufweist, welche entlang der Seitenwand (83) positioniert sind, wobei jeder Ausrichtungsschlitz (88) ausgelegt ist, um mit einer von einer Mehrzahl an Laschen (91) auf dem Endbauteil der Wirbelkörperersatzvorrichtung zusammenzupassen, um dabei das Endplattenbauteil (80) in einer gewissen Position relativ zu dem Endbauteil (90) der Wirbelkörperersatzvorrichtung auszurichten;
wobei die Mehrzahl an Löchern (87) sich **durch** das Endplattenbauteil (80) zwischen der Endoberfläche (82) und der Seitenwand (83) erstrecken; und
eines Verschlussmechanismus (84), wobei der Verschlussmechanismus (84) ausgebildet ist, um das Endplattenbauteil (80) an das Endbauteil (90) **durch** eines von der Mehrzahl an Löchern (87) vor der Implantation der Wirbelkörperersatzvorrichtung innerhalb der Wirbelsäule zu koppeln.

2. Endplattenbauteil (80) nach Anspruch 1, wobei der Verschlussmechanismus (84) eine Madenschraube (85) und eine Öffnung aufweist, wobei die Madenschraube ausgebildet ist, um eines von der Mehrzahl an Löchern (87) innerhalb des Endplattenbauteils zu durchqueren und in einer Öffnung (92) aufgenommen zu werden, welche innerhalb des Endbauteils (90) angeordnet ist, wobei die Madenschraube, wenn ihr Gewinde innerhalb der Öffnung eingreift, sicher das Endplattenbauteil an dem Endbauteil koppelt.

3. Endplattenbauteil (80) nach Anspruch 1, wobei die Endoberfläche (82) eine Mehrzahl an Vorsprüngen (86) aufweist, wobei jeder von der Mehrzahl an Vorsprüngen ausgebildet ist, um sich von der Endoberfläche weg zu erstrecken, um dabei vor der Implantation der Wirbelkörperersatzvorrichtung in das Endplattenbauteil (80) einzugreifen und gegen einen angrenzenden Wirbelkörper zu sichern.

4. Endplattenbauteil (80) nach Anspruch 1, wobei die Endoberfläche (82) des Endplattenbauteils im Wesentlichen senkrecht oder in einem Winkel relativ zu der Seitenwand (83) ausgerichtet ist.

5. Endplattenbauteil (80) nach Anspruch 1, wobei ein äußeres Profil der Endoberfläche (82) mindestens eine von einer vieleckigen Form, einer nicht kreisförmigen Form und einer kreisförmigen Form hat, wobei die Form des äußeren Profils ausgewählt ist, um der Form des angrenzenden Wirbelkörpers zu entsprechen, nach der Implantation der Wirbelkörperersatzvorrichtung innerhalb der Wirbelsäule.

6. Endplattenbauteil (90) nach Anspruch 1, wobei die Endoberfläche (82) des Endplattenbauteils eine Mehrzahl an Oberflächenbestandteilen aufweist, wobei die Oberflächenbestandteile Oberflächenbestandteile in Mikrogröße oder Nanogröße sind.

7. Endplattenbauteil (80) nach Anspruch 1, wobei die Endoberfläche (82) des Endplattenbauteils ausgebildet und mit einer Substanz beschichtet ist, um das biologische Einwachsen von der Endoberfläche in einen angrenzenden Wirbelkörper zu erleichtern, nach der Implantation der Wirbelkörperersatzvorrichtung innerhalb einer Wirbelsäule.

8. Verfahren für den Zusammenbau einer Wirbelkörperersatzvorrichtung (10), wobei das Verfahren **gekennzeichnet ist durch** das Aufweisen:
des Erlangens eines Körperbauteils (30), wobei das Körperbauteil ein länglicher Körper mit einer Innenwand (31) und einer Außenwand (32) ist, und eine erste Endaufnahme (33), eine zweite Endaufnahme (34) und eine Längsachse (72) aufweist, welche sich zwischen der ersten Endaufnahme und der zweiten Endaufnahme davon erstreckt;
des Erlangens eines zentralen Stabbauteils (40), mit einem ersten Gewindeabschnitt (41), einem zweiten Gewindeabschnitt (42) und einer Mittelachse (46), welche sich dazwischen erstreckt, wobei das zentrale Stabbauteil ausgebildet ist, um im Betrieb mit dem Körperbauteil (30) verbunden zu sein;
des Erlangens eines ersten Endbauteils (20) und eines zweiten Endbauteils (20), wobei das erste Endbauteil ausgebildet ist, um innerhalb der ersten Endaufnahme (33) des Körperbauteils (30) positioniert zu sein, um über ein Gewinde in den ersten Gewindeabschnitt (41) des zentralen Stabbauteils (40) einzugreifen, wenn das zentrale Stabbauteil im Betrieb mit dem Körperbauteil verbunden ist, und das zweite Endbauteil (20) ausgebildet ist, um innerhalb der zweiten Endaufnahme (34) des Körperbauteils positioniert zu sein, um über ein Gewinde in den zweiten Gewindeabschnitt (42) des zentralen Stabbauteils einzugreifen, wenn das zentrale Stabbauteil im Betrieb mit dem Körperbauteil verbunden ist; und
des Erlangens zumindest eines Endplattenbauteils (80), wobei das zumindest eine Endplattenbauteil aufweist:
eine Endoberfläche (82), wobei die Endoberfläche (82) ausgebildet ist, um in eine Knochenoberfläche einzugreifen, bei einer Implantation der Wirbelkörperersatzvorrichtung (10) innerhalb einer Wirbelsäule, wobei die Endoberfläche (82) einen Mittelabschnitt, der massiv ist, und eine Mehrzahl an Löchern (87) hat, welche den Mittelabschnitt der Endoberfläche (82) umgeben und sich **durch** das Endplattenbauteil (80) erstrecken;
eine Seitenwand (83), die mit der Endoberfläche (82) verbunden ist, wobei die Endoberfläche (82) ein gesamtes Gebiet aufspannt, welches **durch** die Seitenwand (83) begrenzt ist, und wobei die Seitenwand (83) ausgebildet ist, um einen Einstellungsmechanismus (93) zu haben, wobei der Einstellungsmechanismus die Positionierung des zumindest einen Endplattenbauteils (80) relativ zu mindestens einem von dem ersten Endbauteil (20) und dem zweiten Endbauteil (20) erleichtert, wobei der Einstellungsmechanismus (93) eine Mehrzahl an Ausrichtungsschlitzen (88) aufweist, welche entlang der Seitenwand (83) positioniert sind, wobei jeder Ausrichtungsschlitz (88) ausgelegt ist, um mit einer von einer Mehrzahl an Laschen (91) auf zumindest einem von dem ersten Endbauteil (20) und dem zweiten Endbauteil (20) zusammenzupassen, um dabei das zumindest eine Endplattenbauteil (80) in einer gewissen Position relativ zu dem zumindest einem von dem ersten Endbauteil (20) und dem zweiten Endbauteil (20) auszurichten; und
einen Verschlussmechanismus (84), wobei der Verschlussmechanismus (84) ausgebildet ist, um das zumindest eine Endplattenbauteil (80) an das zumindest eine von dem ersten Endbauteil (20) und dem zweiten Endbauteil (20) zu koppeln, um es dabei dem Endplattenbauteil zu gestatten modular zu sein;
des Anordnens des zentralen Stabbauteils (40) innerhalb einer mittleren Kammer (36) des Körperbauteils (30) und des Verbindens im Betrieb des zentralen Stabbauteils mit dem Körperbauteil;
des in Eingriffbringens, über ein Gewinde, des ersten Gewindeabschnitts (41) des zentralen Stabbauteils (40) mit dem ersten Endbauteil (20) und des in Eingriffbringens, über ein Gewinde, des zweiten Gewindeabschnitts (42) des zentralen Stabbauteils mit dem zweiten Endbauteil (20);
des Verwendens des Einstellungsmechanismus (93), um das zumindest eine Endplattenbauteil (80) in einer gewissen Position relativ zu dem Endbauteil (90) auszurichten; und
des Verwendens des Verschlussmechanismus (84), um das zumindest eine Endplattenbauteil (80) mit zumindest einem von dem ersten Endbauteil (20) und dem zweiten Endbauteil (20) vor der Implantation der Wirbelkörperersatzvorrichtung innerhalb einer Wirbelsäule zu verbinden.

9. Verfahren nach Anspruch 8, wobei das Erlangen zumindest eines Endplattenbauteils (80) ferner den Einstellungsmechanismus (93) aufweist, welcher eine Mehrzahl an Laschen (91) aufweist, welche an einer Endwand von jedem von dem ersten Endbauteil (20) und dem zweiten Endbauteil (20) angeordnet sind und den Ausrichtungsschlitzen (88) entsprechen, welche entlang der Seitenwand (83) des Endplattenbauteils (80) positioniert sind, wobei jeder Ausrichtungsschlitz (88) ausgelegt ist, um mit einer von der Mehrzahl an Laschen (91) zusammenzupassen, und um dabei das Endplattenbauteil (80) in einer gewissen Position relativ zu der Endwand von zumindest einem von dem ersten Endbauteil (20) und dem zweiten Endbauteil (20) und einen Wirbelkörper zu positionieren, nach der Implantation der Wirbelkörperersatzvorrichtung.

10. Verfahren nach Anspruch 8, wobei die Endoberfläche von dem zumindest einen Endplattenbauteils im Wesentlichen normal oder in einem Winkel relativ zu der Seitenwand (83) ausgerichtet ist.

11. Verfahren nach Anspruch 8, wobei ein äußeres Profil der Endoberfläche von dem zumindest einem Endplattenbauteil (80) mindestens eine von einer vieleckigen Form, einer nicht kreisförmigen Form und einer kreisförmigen Form hat, wobei die Form des äußeren Profils ausgewählt wird, um der Form des angrenzenden Wirbelkörpers zu entsprechen, nach der Implantation der Wirbelkörperersatzvorrichtung innerhalb einer Wirbelsäule.

12. Verfahren nach Anspruch 8, wobei die Endoberfläche des zumindest einen Endplattenbauteils (80) eine Mehrzahl an Oberflächenbestandteilen aufweist, wobei die Oberflächenbestandteile in Mikrogröße oder Nanogröße sind.

13. Verfahren nach Anspruch 8, wobei die Endoberfläche von dem zumindest einen Endplattenbauteil (80) ausgebildet und mit einer Substanz beschichtet ist, um das biologische Einwachsen von der Endoberfläche in einen angrenzenden Wirbelkörper zu erleichtern, nach der Implantation der Wirbelkörperersatzvorrichtung innerhalb einer Wirbelsäule.

14. Verfahren nach Anspruch 8, das ferner das Erlangen eines Stützrings (50) aufweist, wobei der Stützring eine Lageroberfläche (51) hat, und ausgebildet ist, um über ein Gewinde in der Innenwand des Körperbauteils (30) einzugreifen, um es dabei der Lageroberfläche zu gestatten das zentrale Stabbauteil (40) zu berühren, wenn das zentrale Stabbauteil im Betrieb innerhalb des Körperbauteils positioniert ist.

15. Verfahren nach Anspruch 14, wobei das Erlangen eines zentralen Stabbauteils (40) ferner einen Zahnradabschnitt (43) aufweist, wobei der Zahnradabschnitt eine verzahnte Oberfläche (44) und eine Stützoberfläche (45) hat, wobei die Stützoberfläche des Zahnradabschnitts ausgebildet ist, um die Lageroberfläche des Stützrings (50) zu berühren, wenn das zentrale Stabbauteil im Betrieb innerhalb des Körperbauteils positioniert ist.

16. Verfahren nach Anspruch 15, wobei das zentrale Stabbauteil (40) eine Mittelachse (46) aufweist, welche sich zwischen dem ersten Gewindeabschnitt (41) und dem zweiten Gewindeabschnitt (42) davon erstreckt, und wobei die Rotationsachse des Zahnradabschnitts (43) im Wesentlichen koaxial zu der Mittelachse des zentralen Stabbauteils ist, wenn dabei der Zahnradabschnitt um die Rotationsachse rotiert wird, rotieren entsprechend der erste und zweite Gewindeabschnitt um die Mittelachse (46) des zentralen Stabbauteils.

17. Verfahren für den Gebrauch von zumindest einem Endplattenbauteil (80) in einer Wirbelkörperersatzvorrichtung (10), wobei das Verfahren **gekennzeichnet ist durch** das Aufweisen:
des Erlangens einer Wirbelkörperersatzvorrichtung (10), wobei die Wirbelkörperersatzvorrichtung ein Körperbauteil (30), ein zentrales Stabbauteil (40) mit einem ersten Gewindeabschnitt (41) und einem zweiten Gewindeabschnitt (42), wobei das zentrale Stabbauteil ausgebildet ist, um im Betrieb mit dem Körperbauteil verbunden zu werden, und ein erstes Endbauteil (20) und ein zweites Endbauteil (20) enthält, wobei das erste Endbauteil ausgebildet ist, um über ein Gewinde in den ersten Gewindeabschnitt (41) des zentralen Stabbauteils (40) einzugreifen und das zweite Endbauteil ausgebildet ist, um über ein Gewinde in den zweiten Gewindeabschnitt (42) des zentralen Stabbauteils einzugreifen;
des Erlangens zumindest eines Endplattenbauteils (80), wobei das zumindest eine Endplattenbauteil eine Endoberfläche (82) aufweist, wobei die Endoberfläche (82) einen Mittelabschnitt, der massiv ist, und eine Mehrzahl an Löchern (87) hat, welche den Mittelabschnitt der Endoberfläche (82) umgeben und sich **durch** das Endplattenbauteil (80) erstrecken, und einer Seitenwand (83), die mit der Endoberfläche verbunden ist, wobei die Seitenwand ausgebildet ist, um einen Einstellungsmechanismus (93) zu haben, wobei der Einstellungsmechanismus (93) die Positionierung von dem zumindest einen Endplattenbauteils (80) relativ zu zumindest einem von dem ersten Endbauteil (20) und dem zweiten Endbauteil (20) erleichtert, und eines Verschlussmechanismus (84), wobei der Verschlussmechanismus (84) ausgebildet ist, um das zumindest eine Endplattenbauteil (80) an zumindest einem von dem ersten Endbauteil (20) und dem zweiten Endbauteil (20) zu koppeln, wobei der Einstellungsmechanismus (93) eine Mehrzahl an Ausrichtungsschlitzen (88) aufweist, welche entlang der Seitenwand (83) positioniert sind, wobei jeder Ausrichtungsschlitz (88) ausgelegt ist, um mit einer der Mehrzahl an Laschen (91) auf zumindest einem von dem ersten Endbauteil (20) und dem zweiten Endbauteil (20) zusammenzupassen, um dabei das zumindest ein Endplattenbauteil (80) in einer gewissen Position relativ zu zumindest einem von dem ersten Endbauteil (20) und dem zweiten Endbauteil (20) auszurichten; und
des Kuppelns des zumindest einen Endplattenbauteils (80) an zumindest einem von dem ersten Endbauteil (20) und dem zweiten Endbauteil (20), wobei der Verschlussmechanismus sicher das zumindest eine Endplattenbauteil mit zumindest einem von dem ersten Endbauteil und dem zweiten Endbauteil verbindet.

## Revendications

1. Elément de plaque de support (80) destiné à être utilisé dans un dispositif de remplacement de corps vertébral (10), l'élément de plaque de support (80) étant **caractérisé en ce qu'**il comprend :
une surface d'extrémité (82), dans lequel la surface d'extrémité (82) est configurée pour mettre en prise une surface osseuse suite à l'implantation du dispositif de remplacement de corps vertébral à l'intérieur d'une colonne vertébrale, la surface d'extrémité (82) ayant une section centrale qui est pleine, et une pluralité de trous (87) entourant la section centrale de la surface d'extrémité (82) et s'étendant à travers l'élément de plaque de support (80) ;
une paroi latérale (83) raccordée à la surface d'extrémité (82) avec la surface d'extrémité (82) qui recouvre toute une surface délimitée par la paroi latérale (83), et avec la paroi latérale (83) qui est configurée pour avoir un mécanisme d'ajustement (93), dans lequel le mécanisme d'ajustement facilite le positionnement de l'élément de plaque de support (80) par rapport à un élément d'extrémité (90) du dispositif de remplacement de corps vertébral, le mécanisme d'ajustement (93) comprenant une pluralité de fentes d'alignement (88) positionnées le long de la paroi latérale (83), dans lequel chaque fente d'alignement (88) est dimensionnée pour se coupler avec l'une d'une pluralité de languettes (91) sur ledit élément d'extrémité du dispositif de remplacement de corps vertébral, orientant ainsi l'élément de plaque de support (80) dans une certaine position par rapport audit élément d'extrémité (90) du dispositif de remplacement de corps vertébral ;
la pluralité de trous (87) s'étendant à travers l'élément de plaque de support (80) entre la surface d'extrémité (82) et la paroi latérale (83) ; et
un mécanisme de verrouillage (84), dans lequel le mécanisme de verrouillage (84) est configuré pour coupler l'élément de plaque de support (80) à l'élément d'extrémité (90) par le biais de l'un de la pluralité de trous (87) avant l'implantation du dispositif de remplacement de corps vertébral à l'intérieur de la colonne vertébrale.

2. Elément de plaque de support (80) selon la revendication 1, dans lequel le mécanisme de verrouillage (84) comprend une vis de pression (85) et une ouverture, dans lequel la vis de pression est configurée pour passer à travers l'un de la pluralité de trous (87) à l'intérieur de l'élément de plaque de support et pour être reçue dans une ouverture (92) disposée à l'intérieur de l'élément d'extrémité (90), la vis de pression, lorsqu'elle est mise en prise par filetage à l'intérieur de l'ouverture, couple, de manière fixe, l'élément de plaque de support à l'élément d'extrémité.

3. Elément de plaque de support (80) selon la revendication 1, dans lequel la surface d'extrémité (82) comprend une pluralité de saillies (86), chacune de la pluralité de saillies est configurée pour s'étendre à distance de la surface d'extrémité, pour mettre ainsi en prise et fixer l'élément de plaque de support (80) contre un corps vertébral adjacent, suite à l'implantation du dispositif de remplacement de corps vertébral.

4. Elément de plaque de support (80) selon la revendication 1, dans lequel la surface d'extrémité (82) de l'élément de plaque de support est orientée sensiblement perpendiculairement ou à un angle par rapport à la paroi latérale (83).

5. Elément de plaque de support (80) selon la revendication 1, dans lequel un profil externe de la surface d'extrémité (82) est au moins l'une parmi une forme polygonale, une forme non circulaire et une forme circulaire, dans lequel la forme du profil externe de la surface d'extrémité est choisie pour correspondre à la forme du corps vertébral adjacent suite à l'implantation du dispositif de remplacement de corps vertébral à l'intérieur de la colonne vertébrale.

6. Elément de plaque de support (90) selon la revendication 1, dans lequel la surface d'extrémité (82) de l'élément de plaque de support comprend une pluralité de caractéristiques de surface, dans lequel les caractéristiques de surface sont des caractéristiques de surface de la taille du micron ou de taille nanométrique.

7. Elément de plaque de support (80) selon la revendication 1, dans lequel la surface d'extrémité (82) de l'élément de plaque de support est configurée et recouverte avec une substance afin de faciliter la croissance naturelle de la surface d'extrémité sur le corps vertébral adjacent suite à l'implantation du dispositif de remplacement de corps vertébral à l'intérieur d'une colonne vertébrale.

8. Procédé pour assembler un dispositif de remplacement de corps vertébral (10), le procédé étant **caractérisé en ce qu'**il comprend les étapes consistant à :
obtenir un élément de corps (30), dans lequel l'élément de corps est un corps allongé ayant une paroi interne (31) et une paroi externe (32), et comprenant un premier réceptacle d'extrémité (33), un second réceptacle d'extrémité (34) et un axe longitudinal (72) s'étendant entre le premier réceptacle d'extrémité et son second réceptacle d'extrémité ;
obtenir un élément de tige centrale (40) ayant une première partie filetée (41), une seconde partie filetée (42) et un axe central (46) s'étendant à travers ce dernier, l'élément de tige centrale étant configuré pour être associé de manière opérationnelle avec l'élément de corps (30) ;
obtenir un premier élément d'extrémité (20) et un second élément d'extrémité (20), dans lequel le premier élément d'extrémité est configuré pour être positionné à l'intérieur du premier réceptacle d'extrémité (33) de l'élément de corps (30) afin de mettre en prise, par filetage, la première partie filetée (41) de l'élément de tige centrale (40) lorsque l'élément de tige centrale est associé de manière opérationnelle avec l'élément de corps, et le second élément d'extrémité (20) est configuré pour être positionné à l'intérieur du second réceptacle d'extrémité (34) de l'élément de corps afin de mettre en prise, par filetage, la seconde partie filetée (42) de l'élément de tige centrale, lorsque l'élément de tige centrale est associé de manière opérationnelle à l'élément de corps ; et
obtenir au moins un élément de plaque de support (80), dans lequel le au moins un élément de plaque de support comprend :
une surface d'extrémité (82), dans lequel la surface d'extrémité (82) est configurée pour mettre en prise une surface osseuse, suite à l'implantation du dispositif de remplacement de corps vertébral (10) à l'intérieur d'une colonne vertébrale, la surface d'extrémité (82) ayant une section centrale qui est pleine, et une pluralité de trous (87) entourant la section centrale de surface d'extrémité (82) et s'étendant à travers l'élément de plaque de support (80) ;
une paroi latérale (83) raccordée à la surface d'extrémité (82) avec la surface d'extrémité (82) qui recouvre toute une surface délimitée par la paroi latérale (83) et étant configurée pour avoir un mécanisme d'ajustement (93), dans lequel le mécanisme d'ajustement facilite le positionnement du au moins un élément de plaque de support (80) par rapport à au moins l'un parmi le premier élément d'extrémité (20) et le second élément d'extrémité (20), le mécanisme d'ajustement (93) comprenant une pluralité de fentes d'alignement (88) positionnées le long de la paroi latérale (83), dans lequel chaque fente d'alignement (88) est dimensionnée pour se coupler avec l'une d'une pluralité de languettes (91) sur au moins l'un parmi le premier élément d'extrémité (20) et le second élément d'extrémité (20), orientant ainsi le au moins un élément de plaque de support (80) dans une certaine position par rapport à au moins l'un parmi le premier élément d'extrémité (20) et le second élément d'extrémité (20) ; et
un mécanisme de verrouillage (84), dans lequel le mécanisme de verrouillage est configuré pour coupler le au moins un élément de plaque de support (80) à au moins l'un parmi le premier élément d'extrémité (20) et le second élément d'extrémité (20), permettant ainsi à la plaque de support d'être modulaire ;
placer l'élément de tige centrale (40) à l'intérieur d'une chambre centrale (36) de l'élément de corps (30) et associer de manière opérationnelle l'élément de tige centrale avec l'élément de corps ;
mettre en prise, par filetage, la première partie filetée (41) de l'élément de tige centrale (40) avec le premier élément d'extrémité (20) et mettre en prise, par filetage, la seconde partie filetée (42) de l'élément de tige centrale avec le second élément d'extrémité (20) ;
utiliser le mécanisme d'ajustement (93) pour orienter le au moins un élément de plaque de support (80) dans une certaine position par rapport audit élément d'extrémité (90) ; et
utiliser le mécanisme de verrouillage (84) pour raccorder le au moins un élément de plaque de support (80) à au moins l'un parmi le premier élément d'extrémité (20) et le second élément d'extrémité (20) avant d'implanter le dispositif de remplacement de corps vertébral à l'intérieur d'une colonne vertébrale.

9. Procédé selon la revendication 8, dans lequel l'étape consistant à obtenir au moins un élément de plaque de support (80) comprend en outre le mécanisme d'ajustement (93) qui comprend une pluralité de languettes (91) qui sont disposées sur une paroi d'extrémité de chacun parmi le premier élément d'extrémité (20) et le second élément d'extrémité (20), et les fentes d'alignement (88) correspondantes étant positionnées le long de la paroi latérale (83) de l'élément de plaque de support (80), dans lequel chaque fente d'alignement (88) est dimensionnée pour se coupler avec l'une de la pluralité de languettes (91) et, orienter ainsi l'élément de plaque de support (80) dans une certaine position par rapport à la paroi d'extrémité d'au moins l'un parmi le premier élément d'extrémité (20) et le second élément d'extrémité (20) et un corps vertébral suite à l'implantation du dispositif de remplacement de corps vertébral.

10. Procédé selon la revendication 8, dans lequel la surface d'extrémité du au moins un élément de plaque de support (80) est orientée sensiblement perpendiculairement ou à un angle par rapport à la paroi latérale (83).

11. Procédé selon la revendication 8, dans lequel un profil externe de la surface d'extrémité du au moins un élément de plaque de support (80) est au moins l'une parmi une forme polygonale, une forme non circulaire et une forme circulaire, dans lequel la forme de profil externe de la surface d'extrémité est choisie pour correspondre à la forme du corps vertébral adjacent suite à l'implantation du dispositif de remplacement de corps vertébral à l'intérieur d'une colonne vertébrale.

12. Procédé selon la revendication 8, dans lequel la surface d'extrémité du au moins un élément de plaque de support (80) comprend une pluralité de caractéristiques de surface avec les caractéristiques de surface qui sont de la taille du micron ou de taille nanométrique.

13. Procédé selon la revendication 8, dans lequel la surface d'extrémité du au moins un élément de plaque de support (80) est configurée et recouverte avec une substance pour faciliter la croissance naturelle de la surface d'extrémité sur le corps vertébral adjacent suite à l'implantation du dispositif de remplacement de corps vertébral à l'intérieur d'une colonne vertébrale.

14. Procédé selon la revendication 8, comprenant en outre l'étape consistant à obtenir une bague de support (50), dans lequel la bague de support a une surface d'appui (51) et est configurée pour mettre en prise, par filetage, la paroi interne de l'élément de corps (30), permettant ainsi à la surface d'appui de venir en contact avec l'élément de tige centrale (40) lorsque l'élément de tige centrale est positionné de manière opérationnelle à l'intérieur de l'élément de corps.

15. Procédé selon la revendication 14, dans lequel l'étape consistant à obtenir un élément de tige centrale (40) comprend en outre une partie de roue dentée (43), la partie de roue dentée ayant une surface dentée (44) et une surface de support (45), la surface de support de la partie de roue dentée étant configurée pour venir en contact avec la surface d'appui de la bague de support (50) lorsque l'élément de tige centrale est positionné de manière opérationnelle à l'intérieur de l'élément de corps.

16. Procédé selon la revendication 15, dans lequel l'élément de tige centrale (40) comprend un axe central (46) s'étendant entre la première partie filetée (41) et sa seconde partie filetée (42), et dans lequel l'axe de rotation de la partie de roue dentée (43) est sensiblement coaxial par rapport à l'axe central de l'élément de tige centrale, ainsi, lorsque la partie de roue dentée est entraînée en rotation autour de l'axe de rotation, les première et seconde parties filetées tournent de manière correspondante autour de l'axe central (46) de l'élément de tige centrale.

17. Procédé pour utiliser au moins un élément de plaque de support (80) dans un dispositif de remplacement de corps vertébral (10), le procédé étant **caractérisé en ce qu'**il comprend les étapes consistant à :
obtenir un dispositif de remplacement de corps vertébral (10), le dispositif de remplacement de corps vertébral comprenant un élément de corps (30), un élément de tige centrale (40) ayant une première partie filetée (41) et une seconde partie filetée (42), l'élément de tige centrale étant configuré pour être associé de manière opérationnelle à l'intérieur de l'élément de corps et d'un premier élément d'extrémité (20) et d'un second élément d'extrémité (20), le premier élément d'extrémité étant configuré pour mettre en prise, par filetage, la première partie filetée (41) de l'élément de tige centrale (40) et le second élément d'extrémité étant configuré pour mettre en prise, par filetage, la seconde partie filetée (42) de l'élément de tige centrale ;
obtenir au moins un élément de plaque de support (80), le au moins un élément de plaque de support comprenant une surface d'extrémité (82), la surface d'extrémité (82) ayant une section centrale qui est pleine, et une pluralité de trous (87) entourant la section centrale de surface d'extrémité (82) et s'étendant à travers l'élément de plaque de support (80), et une paroi latérale (83) raccordée à la surface d'extrémité, avec la paroi latérale qui est configurée pour avoir un mécanisme d'ajustement (93), dans lequel le mécanisme d'ajustement facilite le positionnement du au moins un élément de plaque de support (80) par rapport à au moins l'un parmi le premier élément d'extrémité (20) et le second élément d'extrémité (20), et un mécanisme de verrouillage (84), dans lequel le mécanisme de verrouillage (84) est configuré pour coupler le au moins un élément de plaque de support (80) à au moins l'un parmi le premier élément d'extrémité (20) et le second élément d'extrémité (20), le mécanisme d'ajustement (93) comprenant une pluralité de fentes d'alignement (88) positionnées le long de la paroi latérale (83), dans lequel chaque fente d'alignement (88) est dimensionnée pour se coupler avec l'une d'une pluralité de languettes (91) sur au moins l'un parmi le premier élément d'extrémité (20) et le second élément d'extrémité (20), orientant ainsi le au moins un élément de plaque de support (80) dans une certaine position par rapport à au moins l'un parmi le premier élément d'extrémité (20) et le second élément d'extrémité (20) ; et
coupler le au moins un élément de plaque de support (80) à au moins l'un parmi le premier élément d'extrémité (20) et le second élément d'extrémité (20), le mécanisme de verrouillage raccordant, de manière fixe, le au moins un élément de plaque de support à au moins l'un parmi le premier élément d'extrémité et le second élément d'extrémité.
